# EUROPEAN PATENT APPLICATION

(11) **EP 4 628 569 A1**
(43) Date of publication of application: **08.10.2025**
(21) Application number: 23897646.8
(22) Date of filing: 22.11.2023
(51) Int. Cl.: C12M 1/00, C12M 3/00, C12N 5/071

(54) **CELL SHEET PRODUCTION DEVICE AND STERILE CLOSED CIRCUIT FOR CELL SHEET PRODUCTION DEVICE**

(30) Priority: 28.11.2022 JP 2022189691
(71) Applicant: Nitta Corporation, Osaka-shi, Osaka 556-0022 (JP)
(72) Inventor: HATSUDA, Masahiro, Osaka-shi, Osaka 556-0022 (JP); ISHII, Daiki, Osaka-shi, Osaka 556-0022 (JP); KAMENO, Shunsuke, Osaka-shi, Osaka 556-0022 (JP)
(74) Representative: Gille Hrabal Partnerschaftsgesellschaft mbB Patentanwälte
(86) International application number: PCT/JP2023/041990
(87) International publication number: WO 2024/116998

(57) **Abstract**

Provided are a small cell sheet production device capable of producing a cell sheet by an automatic operation, and a sterile closed circuit for a cell sheet production device used in the cell sheet production device. A cell sheet production device 1, to which a sterile closed circuit for a cell sheet production device 100 is to be attached, the sterile closed circuit including a sheet forming container 15 provided with a sheet forming solution tank having a plane substrate for cell adhesion for producing a cell sheet 80, a first pipe 68 that injects a cell suspension in which cells are suspended into the sheet forming container 15, second pipes 66a and 67 that inject a culture solution for medium exchange, and a third pipe 72 that discharges an unnecessary liquid component, the cell sheet production device 1 including a concentration unit (centrifuge 11) that concentrates the cell suspension. In the cell sheet production device 1, the cell suspension is concentrated, the concentrated cell suspension is injected into the sheet forming container 15, the cells are seeded on a plane substrate for cell adhesion at a cell seeding density of 3,000 cells/mm² or more to produce a cell sheet on the plane substrate for cell adhesion.

## Description

### Technical Field

The present invention relates to a cell sheet production device and a sterile closed circuit for a cell sheet production device.

### Background Art

Age-related macular degeneration includes an exudative type and an atrophic type, and a treatment for a mild exudative type thereof includes methods such as antiangiogenic therapy, photodynamic therapy, and laser photocoagulation. However, in the case of an exudative type or atrophic type which has progressed to show severe atrophy and deficiency of retinal pigment epithelium (RPE), there is a concern that the aforementioned means fails to show the effectiveness of treatment. As an effective treatment method in this case, a method of transplanting a sheet of iPS cell-derived RPE cells of a patient has been developed.

Production of a cell sheet for this treatment is manually performed by a professional engineer using a cell processing center (CPC) having a safety cabinet or an advanced clean room, dedicated equipment such as an isolator system, or the like.

Patent Literature 1 discloses a method for producing an RPE cell sheet, the method including seeding RPE cells on a collagen gel and culturing the RPE cells, forming a cell sheet composed of the RPE cells, then degrading the collagen gel with collagenase, and detaching the cell sheet composed of the RPE cells.

Patent Literature 2 discloses a method for producing a cell sheet.

### Citation List

### Patent Literature

Patent Literature 1: PCT International Publication No. WO2012/115244
Patent Literature 2: JP5867926B

### Summary of Invention

### Technical Problem

In a case where a cell sheet is produced using a CPC, a safety cabinet or a clean room of the CPC is an open system, and even when the cleanliness of the clean room is increased, contamination such as contact of bacteria with cells may occur. In addition, since cell culture and the production of the cell sheet are all manually performed, variation in quality is likely to occur, and in a case of poor quality, the production of the cell sheet may fail.

In a case where a cell sheet is produced by an isolator system, the isolator system is a closed system and may be automated, but high cost is required for the automation. In addition, in order to make a cell sheet using cells of a patient himself/herself, it is necessary to execute pipe washing and stage change of an automation device individually for each patient, which is inefficient.

Since the center and the equipment in either case are large, it is necessary to produce the cell sheet at a place different from a place where the cells are separated and transplanted. In this case, the produced cell sheet is transported to a place where a transplantation treatment is performed, but the cell sheet may be damaged due to external factors such as vibration and impact received in the transport process, or the passage of time.

For this reason, it is required to easily culture cells using a small and inexpensive device without requiring a special skill and equipment, and to automatically perform the process up to formation of a sheet or culture and formation of a three-dimensional tissue.

Therefore, an object of the present invention is to provide a small cell sheet production device capable of producing a cell sheet by automatically operating steps such as mixing, separating, dispensing, and seeding of a cell suspension, a culture solution, and the like, which are raw materials, under optimum conditions without a manual work, and a sterile closed circuit for a cell sheet production device used in the cell sheet production device.

### Solution to Problem

A cell sheet production device according to the present invention, to which a sterile closed circuit for a cell sheet production device is to be attached, the sterile closed circuit including a sheet forming container provided with a sheet forming solution tank having a plane substrate for cell adhesion for producing a cell sheet, a first pipe that is connected to the sheet forming container and injects a cell suspension in which cells are suspended into the sheet forming container, a second pipe that is connected to the sheet forming container and injects a culture solution for medium exchange into the sheet forming container, and a third pipe that is connected to the sheet forming container and discharges an unnecessary liquid component inside the sheet forming container, the cell sheet production device including a concentration unit that is connected to the sheet forming container and concentrates the cell suspension. In the cell sheet production device, a concentration of the cell suspension is concentrated in the concentration unit, the concentrated cell suspension is injected into the sheet forming container through the first pipe, the cells are seeded on the plane substrate for cell adhesion at a cell seeding density of 3,000 cells/mm² or more, the unnecessary liquid component is discharged from the third pipe, and the culture solution for medium exchange is injected from the second pipe, thereby producing a cell sheet on the plane substrate for cell adhesion.

A cell sheet production device according to the present invention, to which a sterile closed circuit for a cell sheet production device is to be attached, the sterile closed circuit including a sheet forming container provided with a sheet forming solution tank having a plane substrate for cell adhesion for producing a cell sheet, a first pipe that is connected to the sheet forming container and injects a cell suspension in which cells are suspended into the sheet forming container, a second pipe that is connected to the sheet forming container and injects a culture solution for medium exchange into the sheet forming container, and a third pipe that is connected to the sheet forming container and discharges an unnecessary liquid component inside the sheet forming container, the cell sheet production device including: a concentration unit that is connected to the sheet forming container and concentrates the cell suspension; a plurality of switching valves provided in a pipe connected to the sheet forming container, the pipe including the first pipe, the second pipe, and the third pipe; and a liquid transferring mechanism that controls switching of the switching valves, and adjusts a pressure inside the sterile closed circuit for a cell sheet production device via a sterile filter, and transfers a liquid inside the sterile closed circuit for a cell sheet production device based on a difference in the pressure. In the cell sheet production device, a concentration of the cell suspension is concentrated in the concentration unit, the concentrated cell suspension is injected into the sheet forming container through the first pipe, the cells are seeded on the plane substrate for cell adhesion at a cell seeding density of 3,000 cells/mm² or more, the unnecessary liquid component is discharged from the third pipe, and the culture solution for medium exchange is injected from the second pipe, thereby producing a cell sheet on the plane substrate for cell adhesion.

A sterile closed circuit for a cell sheet production device according to the present invention, the sterile closed circuit including: a sheet forming container provided with a sheet forming solution tank having a plane substrate for cell adhesion for producing a cell sheet; a first pipe that is connected to the sheet forming container and injects a cell suspension in which cells are suspended into the sheet forming container; a second pipe that is connected to the sheet forming container and injects a culture solution for medium exchange into the sheet forming container; and a third pipe that is connected to the sheet forming container and discharges an unnecessary liquid component inside the sheet forming container. In the sterile closed circuit for a cell sheet production device, a concentration unit that concentrates the cell suspension is connected to the sheet forming container, and a concentration of the cell suspension is concentrated in the concentration unit, the concentrated cell suspension is injected into the sheet forming container through the first pipe, the cells are seeded on the plane substrate for cell adhesion at a cell seeding density of 3,000 cells/mm² or more, the unnecessary liquid component is discharged from the third pipe, and the culture solution for medium exchange is injected from the second pipe, thereby producing a cell sheet on the plane substrate for cell adhesion.

A sterile closed circuit for a cell sheet production device according to the present invention is a sterile closed circuit for a cell sheet production device to be attached to a cell sheet production device to produce a cell sheet, the sterile closed circuit including: a sheet forming container provided with a sheet forming solution tank having a plane substrate for cell adhesion for producing a cell sheet; a first pipe that is connected to the sheet forming container and injects a cell suspension in which cells are suspended into the sheet forming container; a second pipe that is connected to the sheet forming container and injects a culture solution for medium exchange into the sheet forming container; and a third pipe that is connected to the sheet forming container and discharges an unnecessary liquid component inside the sheet forming container. In the sterile closed circuit for a cell sheet production device, a concentration unit that concentrates the cell suspension is connected to the sheet forming container, a plurality of switching valves are provided in a pipe connected to the sheet forming container, the pipe including the first pipe, the second pipe, and the third pipe, a liquid transferring mechanism controls switching of the switching valves, and adjusts a pressure inside the sterile closed circuit for a cell sheet production device via a sterile filter, and transfers a liquid inside the sterile closed circuit for a cell sheet production device based on a difference in the pressure is provided, and a concentration of the cell suspension is concentrated in the concentration unit, the concentrated cell suspension is injected into the sheet forming container through the first pipe, the cells are seeded on the plane substrate for cell adhesion at a cell seeding density of 3,000 cells/mm² or more, the unnecessary liquid component is discharged from the third pipe, and the culture solution for medium exchange is injected from the second pipe, thereby producing a cell sheet on the plane substrate for cell adhesion.

### Advantageous Effects of Invention

According to the cell sheet production device of the present invention, it is possible to provide a small cell sheet production device capable of producing a cell sheet by an automatic operation.

According to the sterile closed circuit for a cell sheet production device of the present invention, it is possible to provide a circuit suitable for use in a small cell sheet production device capable of producing a cell sheet by an automatic operation.

### Brief Description of Drawings

Fig. 1 schematically shows a configuration of a cell sheet production device according to an embodiment.
Fig. 2 schematically shows a configuration of the cell sheet production device in Fig. 1, excluding a sterile closed circuit for a cell sheet production device, a raw material container, and pipes connecting the sterile closed circuit for a cell sheet production device and the raw material container.
Fig. 3 schematically shows a configuration of the sterile closed circuit for a cell sheet production device, the raw material container, and the pipes connecting the sterile closed circuit for a cell sheet production device and the raw material container in the cell sheet production device in Fig. 1.
Fig. 4 schematically shows a configuration of the sterile closed circuit for a cell sheet production device attached to the cell sheet production device in Fig. 1.
Fig. 5 schematically shows a configuration of the raw material container incorporated in the cell sheet production device in Fig. 1.
Fig. 6A shows a top view of a sheet forming container incorporated in the cell sheet production device in Fig. 1.
Fig. 6B shows a side view of the sheet forming container in Fig. 6A.
Fig. 7 shows a cross-sectional view of a part of the sheet forming container incorporated in the cell sheet production device in Fig. 1.
Fig. 8A shows a top view of a configuration in which the sheet forming container of the cell sheet production device in Fig. 1 is attached to a shaking device.
Fig. 8B shows a side view of the configuration in which the sheet forming container in Fig. 8A is attached to the shaking device.
Fig. 9 shows a schematic diagram illustrating how a membrane composed of an extracellular matrix is formed on a surface of a cell sheet.
Fig. 10 schematically shows a configuration of a sheet forming container according to a first example.
Fig. 11 schematically shows a configuration of raw material containers according to a second example.
Fig. 12 schematically shows a configuration of raw material containers according to a third example.
Fig. 13 schematically shows a configuration of raw material containers according to a fourth example.
Fig. 14 shows a temporal change in acceleration according to a sixth example.
Fig. 15 shows a temporal change in acceleration according to a comparative example.
Fig. 16 shows a temporal change in angle according to the sixth example.
Fig. 17 shows a temporal change in angle according to the comparative example.
Fig. 18 schematically shows a configuration of a sterile closed circuit for a cell sheet production device, a raw material container, and pipes connecting the sterile closed circuit for a cell sheet production device and the raw material container in a cell sheet production device according to a seventh example.

### Description of Embodiments

Hereinafter, an embodiment of the present invention will be described. The embodiment described below is merely an example, and appropriate modifications can be made within a range apparent to those skilled in the art.

### <Embodiment>

### (Configuration of Cell Sheet Production Device)

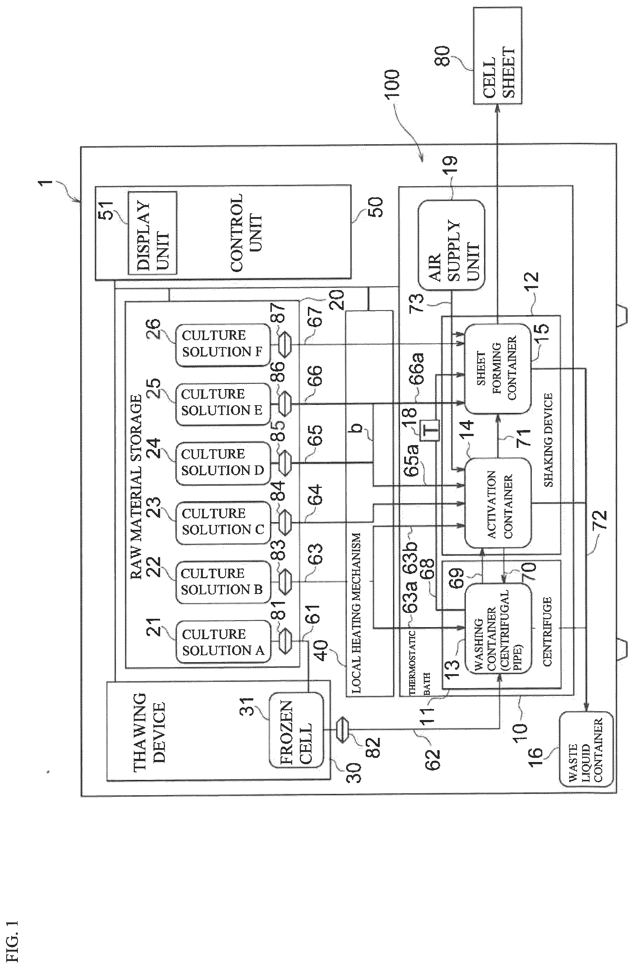

Fig. 1 shows a schematic configuration diagram of a cell sheet production device 1 of the present embodiment. As shown in Fig. 1, the cell sheet production device 1 is configured by attaching a sterile closed circuit for a cell sheet production device 100 thereto. Hereinafter, the cell sheet production device 1 will be described separately for a configuration including the sterile closed circuit for a cell sheet production device 100, the raw material container, and the pipes connecting the sterile closed circuit for a cell sheet production device 100 and the raw material container, and a configuration excluding those.

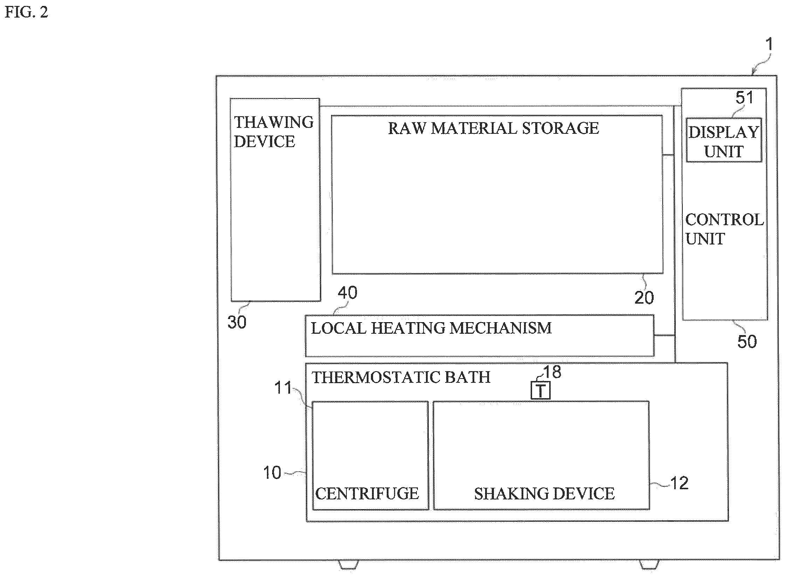

Fig. 2 schematically shows a configuration of the cell sheet production device 1 in Fig. 1, excluding the sterile closed circuit for a cell sheet production device 100, the raw material container, and the pipes connecting the sterile closed circuit for a cell sheet production device 100 and the raw material container. As shown in Fig. 2, the configuration of the cell sheet production device 1 excluding the sterile closed circuit for a cell sheet production device 100, the raw material container, and the pipes connecting the sterile closed circuit for a cell sheet production device 100 and the raw material container includes a thermostatic bath 10, a centrifuge 11, a shaking device 12, an inspection unit 18, a raw material storage 20, a thawing device 30, a local heating mechanism 40, and a control unit 50 provided with a display unit 51.

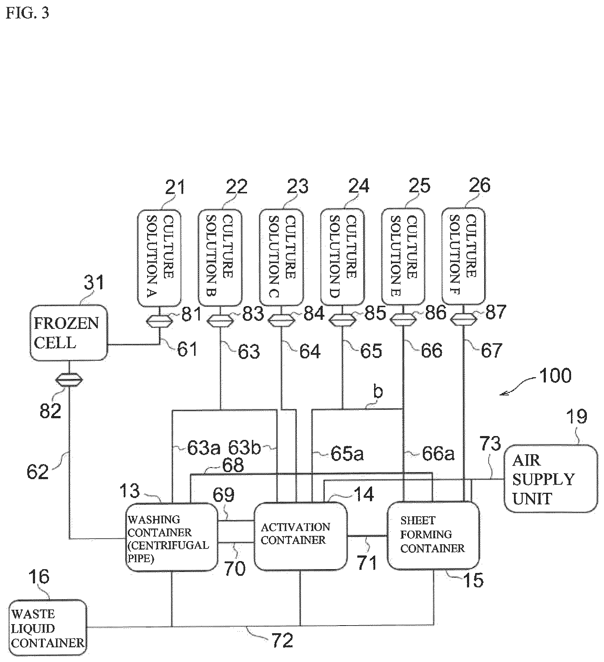

Fig. 3 schematically shows a configuration of the sterile closed circuit for a cell sheet production device 100, the raw material container, and the pipes connecting the sterile closed circuit for a cell sheet production device 100 and the raw material container in the cell sheet production device 1 in Fig. 1. As shown in Fig. 3, the configuration of the sterile closed circuit for a cell sheet production device 100, the raw material container, and the pipes connecting the sterile closed circuit for a cell sheet production device 100 and the raw material container includes a washing container 13, an activation container 14, a sheet forming container 15, a waste liquid container 16, a raw material container 21 for accommodating a culture solution A, a raw material container 22 for accommodating a culture solution B, a raw material container 23 for accommodating a culture solution C, a raw material container 24 for accommodating a culture solution D, a raw material container 25 for accommodating a culture solution E, and a raw material container 26 for accommodating a culture solution F. Further, when the cell sheet production device 1 is used, a frozen cell preservation container 31 for accommodating frozen cells is incorporated, and the drawing shows a state in which the frozen cell preservation container 31 is incorporated.

The raw material container 21 and the frozen cell preservation container 31 are connected through a pipe 61 via a sterile connector 81. The frozen cell preservation container 31 and the washing container 13 are connected through a pipe 62 via a sterile connector 82. A pipe 63 is connected to the raw material container 22 via the sterile connector 83, the pipe 63 is branched into two pipes of a pipe 63a and a pipe 63b, the pipe 63a is connected to the washing container 13, and the pipe 63b is connected to the activation container 14. The raw material container 23 and the activation container 14 are connected through a pipe 64 via a sterile connector 84. The raw material container 24 and the activation container 14 are connected through a pipe 65 and a pipe 65a via a sterile connector 85. The raw material container 25 and the sheet forming container 15 are connected through a pipe 66 and a pipe 66a via a sterile connector 86. Here, the pipe 65 and the pipe 65a, as well as the pipe 66 and the pipe 66a are connected to each other through a pipe b, the raw material container 24 is connected to the sheet forming container 15 through the pipe 65, the pipe b, and the pipe 66a, and the raw material container 25 is connected to the activation container 14 through the pipe 66, the pipe b, and the pipe 65a. The raw material container 26 and the sheet forming container 15 are connected through a pipe 67 via a sterile connector 87. The sterile connectors 81, 82, 83, 84, 85, 86, and 87 are shown in the drawing in a state of being connected after the sterile closed circuit for a cell sheet production device 100 is attached. Before the sterile closed circuit for a cell sheet production device 100 is attached, the sterile connectors 81, 82, 83, 84, 85, 86, and 87 are in a non-connected state.

In the sterile closed circuit for a cell sheet production device 100, the washing container 13, the activation container 14, the sheet forming container 15, the pipes connecting those containers, and the waste liquid container 16 maintain a closed system, and are subjected to sterilization in advance before use.

The washing container 13 and the sheet forming container 15 are connected through a pipe 68. The washing container 13 and the activation container 14 are connected through a pipe 69 and a pipe 70, respectively. The activation container 14 and the sheet forming container 15 are connected through a pipe 71. The washing container 13, the activation container 14, and the sheet forming container 15 are connected to the waste liquid container 16 through a pipe 72, and are configured to discharge unnecessary liquid components inside the washing container 13, the activation container 14, and the sheet forming container 15.

In the above-mentioned configuration, the pipe 71 connecting the activation container 14 and the sheet forming container 15 is a pipe for injecting a cell suspension in which cells are suspended into the sheet forming container 15. The pipe 65, the pipe b, and the pipe 66a connecting the raw material container 24 and the sheet forming container 15, the pipe 66 and the pipe 66a connecting the raw material container 25 and the sheet forming container 15, and the pipe 67 connecting the raw material container 26 and the sheet forming container 15 are each a pipe for injecting a culture solution for medium exchange into the sheet forming container 15. The pipe 72 connecting the sheet forming container 15 and the waste liquid container 16 is a pipe for discharging the unnecessary liquid component inside the sheet forming container 15. The above-mentioned pipes connected to the sheet forming container 15 may be provided independently of each other, and some or all of the pipes can be shared with a pipe having another function by providing a switching unit to enable switching.

The sheet forming container 15 is directly connected to an air supply unit 19 via an air supply pipe 73 to which a sterile filter is attached, or indirectly connected thereto via another tube. Air necessary for producing a cell sheet from the air supply unit 19 can be directly or indirectly injected into the sheet forming container 15. The sterile filter is attached to the air supply pipe 73, so that a sterile environment of air for culture in the thermostatic bath 10 can be maintained.

Each of the above-mentioned pipes has an inner diameter φ of 1 mm to 10 mm, for example. The washing container 13 has a capacity of, for example, 5 ml to 100 ml. The activation container 14 has a capacity of, for example, 50 ml to 1,000 ml.

As shown in Fig. 1, the washing container 13, the activation container 14, and the sheet forming container 15 are accommodated in the thermostatic bath 10. The thermostatic bath 10 is provided to keep a temperature constant in a range of 34°C to 40°C, for example. The washing container 13 is, for example, a centrifugal pipe, and is attached to the centrifuge 11 or the like. The activation container 14 and the sheet forming container 15 are attached to the shaking device 12. In the drawings, the centrifuge 11 and the shaking device 12 are shown as being included in the thermostatic bath 10, but the present invention is not limited thereto, and the centrifuge 11 and the shaking device 12 may not be included therein.

In the present embodiment, the washing container 13 and the activation container 14 are separate containers, but may be integrated as a washing activation container. The washing activation container is appropriately attached to the centrifuge and the shaking device.

The raw material containers 21, 22, 23, 24, 25, and 26 are stored in the raw material storage 20. The raw material storage 20 is provided to keep a temperature constant in a range of 2°C to 10°C, for example.

The frozen cell preservation container 31 is attached to the thawing device 30 when the cell sheet production device 1 is used. The thawing device 30 is provided so as to thaw frozen cells at a temperature in a range of 34°C to 40°C, for example.

A cell suspension is obtained from the frozen cells preserved in the frozen cell preservation container 31, and is formed into a sheet by the cell sheet production device 1 of the present embodiment. Cells to be cultured are not particularly limited as long as the cells can form a cell aggregate by culture, and examples thereof include cells having adhesiveness. Examples of the cells having adhesiveness include RPE cells, liver cancer cells, hepatoblastoma cells, hepatoma cells, hepatocytes which are parenchymal cells of the liver, Kupffer cells, endothelial cells such as vascular endothelial cells and corneal endothelial cells, fibroblasts, osteoblasts, crushed bone cells, periodontium-derived cells, epidermal cells such as epidermal keratinocytes, epithelial cells such as tracheal epithelial cells, gastrointestinal epithelial cells, cervical epithelial cells, and corneal epithelial cells, mammary gland cells, pericytes, muscle cells such as smooth muscle cells and cardiac muscle cells, renal cells, pancreatic islets of Langerhans cells, neurons such as peripheral nerve cells and optic nerve cells, chondrocytes, osteocytes, or stem cells, embryonic stem cells (ES cells), and induced pluripotent stem cells (iPS cells). The stem cells are, for example, bone marrow anaplastic mesenchymal stem cells, hematopoietic stem cells, vascular stem cells, neural stem cells, small intestine stem cells, adipose stem cells, skin stem cells, periodontium stem cells, ciliary body stem cells, limbus stem cells, visceral stem cells, and the like. The cell suspension may contain only one type of cell or a plurality of types of cells. The cell suspension containing a plurality of types of cells can be obtained by, for example, preparing a plurality of frozen cells, thawing each of the frozen cells, and mixing the frozen cells.

The cell suspension is concentrated by the centrifuge 11 or the like. The number of cells per unit volume is referred to as a "cell concentration" or a "cell suspension concentration". The cell suspension is preferably concentrated such that the cell concentration is higher than 5.0 × 10⁵ cells/mL. The cell concentration is monitored by the inspection unit 18 that executes an optical inspection.

The centrifuge 11 is used as a concentration unit that concentrates a cell density of the cell suspension. The concentration unit is not limited to the centrifuge, and may be a concentration unit using a hollow fiber filter or a concentration unit having another configuration.

The concentrated cell suspension is transferred to the sheet forming container 15, and the cells contained in the cell suspension are precipitated and seeded on the plane substrate for cell adhesion provided inside the sheet forming container 15. The number of cells per unit area is referred to as a "cell density" or a "cell seeding density". The higher the cell suspension concentration, the higher the cell seeding density. Cells seeded at a high density are used to produce a cell sheet. The above-mentioned "high density" refers to a density equal to or higher than a cell density observed in a normal tissue derived from prepared cells. An upper limit of the cell density can be appropriately determined by those skilled in the art, and is, for example, a density that does not induce death due to adhesion between the cells. The cell density observed in a normal tissue varies depending on a type of tissue, and is, for example, about 3,000 cells/mm² in a corneal endothelium and about 4,000 cells/mm² in a retinal pigment epithelial cell. The cell density concentrated to a high density is, for example, about 1 time to 100 times, preferably about 1.2 times to 50 times, more preferably about 2.5 times to 30 times, and particularly preferably about 5 times to 10 times the cell density observed in a normal tissue. For example, the cell density is 3,000 cells/mm² or more in the case of corneal endothelium, and 4,000 cells/mm² or more in the case of the retinal pigment epithelial cell. In either case, the cell density is preferably 5,000 cells/mm² or more and 200,000 cells/mm² or less, more preferably 10,000 cells/mm² or more and 120,000 cells/mm² or less, and particularly preferably 20,000 cells/mm² or more and 40,000 cells/mm² or less. In a case where the cell density observed in a normal tissue is smaller than that of the corneal endothelium or the retinal pigment epithelial cell, a range smaller than the above-mentioned cell density can be applied. In a case where the cell density observed in a normal tissue is larger than that of the corneal endothelium or the retinal pigment epithelial cell, a range larger than the above-mentioned cell density can be applied.

The local heating mechanism 40 heats, for example, at least some of the pipes 63, 63a, 63b, 64, 65, 65a, 66, 66a, 67, and b. The local heating mechanism 40 is provided to keep a temperature constant in a range of 34°C to 40°C, for example.

The control unit 50 controls, for example, temperatures of the thermostatic bath 10, the raw material storage 20, the thawing device 30, and the local heating mechanism 40. Setting values and measured values of the temperatures of the thermostatic bath 10, the raw material storage 20, the thawing device 30, and the local heating mechanism 40 are displayed on the display unit 51.

A tube (pellistor) pump is provided in the raw material containers 21, 22, 23, 24, 25, and 26 of the raw material storage 20 or in the pipes connected to those containers, and the control unit 50 is provided to be able to control a flow rate, a timing, and the like of each raw material such as the culture solution supplied from the raw material containers 21, 22, 23, 24, 25, and 26 of the raw material storage 20. Setting values and the like of the flow rate and the timing of each raw material supplied are displayed on the display unit 51. For example, regarding the supply of the culture solution A to the frozen cell preservation container 31 or the washing container 13, the supply of the culture solution B to the washing container 13 or the activation container 14, the supply of the culture solution C to the activation container 14, the supply of the culture solution D to the activation container 14 or the sheet forming container 15, the supply of the culture solution E to the activation container 14 or the sheet forming container 15, and the supply of the culture solution F to the sheet forming container 15, the flow rate and the timing thereof are controlled together with the timing of the production of the cell suspension, the transfer of the cell suspension, and the production of the cell sheet.

A tube (pellistor) pump is provided in the frozen cell preservation container 31, the washing container 13, the activation container 14, the sheet forming container 15, or in the pipes connected to those containers, and the control unit 50 is provided to be able to control the transfer of the culture solution or the cell suspension between the containers.

In order to reduce the number of components and simplify a work of attaching and detaching a closed circuit to and from a main body of the cell sheet production device 1, a pressure control unit may be connected to each of the raw material containers 21, 22, 23, 24, 25, and 26, the frozen cell preservation container 31, the washing container 13, the activation container 14, and the sheet forming container 15 to generate a pressure difference between the containers and transfer a liquid. The pressure control unit executes pressurization by supply of air and depressurization by suction. Each of the raw material containers 21, 22, 23, 24, 25, and 26, the frozen cell preservation container 31, the washing container 13, the activation container 14, and the sheet forming container 15 are connected to the pressure control unit by, for example, a pipe equipped with a sterile filter. Each pipe connected to each of the containers is provided with a switching valve, for example, a pinch valve. The pressure control unit is provided so as to be able to control switching of the switching valve, for example, opening and closing of the pinch valve, and at the same time, adjust a pressure inside each of the raw material containers 21, 22, 23, 24, 25, and 26, the frozen cell preservation container 31, the washing container 13, the activation container 14, and the sheet forming container 15, and the pipes connected to those containers. The adjustment of the pressures inside each of the containers and the pipes is executed by, for example, pressurization by supply of air or depressurization by suction. By switching the switching valve and adjusting the pressures inside each of the containers and the pipes, a pressure difference is generated between specific containers, and the culture solution or the cell suspension is transferred between the containers. The pressure control unit may also be provided in the control unit 50, or may be provided separately from the control unit 50.

In the cell sheet production device 1, a transfer device is also provided in the pipe 72 for waste liquid, and is configured to discharge the waste liquid from the washing container 13, the activation container 14, and the sheet forming container 15 to the waste liquid container 16.

In the present embodiment, after the cell suspension is injected into the sheet forming container 15, the culture solution such as the culture solution D is controlled to be injected therein.

In order to wash the washing container 13, the activation container 14, and the sheet forming container 15 and prevent those from being dried, the culture solution may be supplied from the raw material containers 22, 23, 25, and 26 to the washing container 13, the activation container 14, and the sheet forming container 15 at a preferred timing.

The control unit 50 is connected to the centrifuge 11 and the shaking device 12 and controls driving of the centrifuge 11 and a shaking operation of the shaking device. A setting value of the driving of the centrifuge 11, a setting value of the shaking operation of the shaking device, and the like are displayed on the display unit 51.

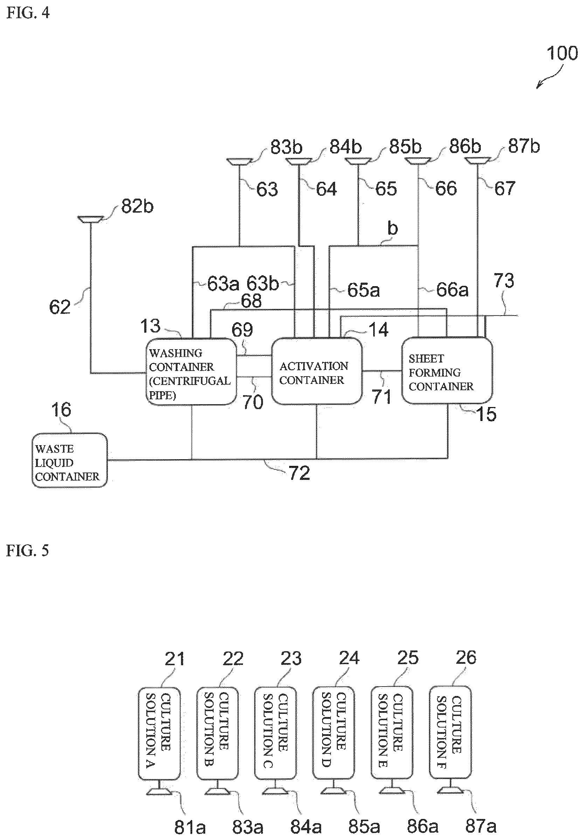

Fig. 4 schematically shows a configuration of the sterile closed circuit for a cell sheet production device 100 attached to the cell sheet production device 1 in Fig. 1. The sterile closed circuit for a cell sheet production device 100 includes the washing container 13, the activation container 14, the sheet forming container 15, and the waste liquid container 16, and is provided with the pipes 62, 63, 63a, 63b, 64, 65, 65a, 66, 66a, b, 67, 68, 69, 70, 71, and 72 connected to those containers. The sterile closed circuit for a cell sheet production device 100 in Fig. 4 shows a state before being attached to the cell sheet production device 1, and sterile connectors 82b, 83b, 84b, 85b, 86b, and 87b respectively constituting the sterile connectors 82, 83, 84, 85, 86, and 87 show a non-connected state.

The cell sheet produced in the sheet forming container 15 is easily detachable from the container without a tool. For example, a groove portion having a depth of 0.1 mm to 0.5 mm is provided on an outer periphery of a side surface of the sheet forming container 15, and the completed cell sheet can be easily detached without a tool by manually applying a force in a direction of separating from an upper portion and a lower portion of the groove.

The pipes 62, 63, 64, 65, 66, 67, and 72 connected to the washing container 13 may be configured to be able to be cut by thermal welding, or a sterile cutting connector may be attached in advance such that the sterile closed circuit can be separated and transported while maintaining a closed system of the sterile closed circuit for a cell sheet production device 100.

Fig. 5 schematically shows a configuration of the raw material containers 21, 22, 23, 24, 25, and 26 incorporated in the cell sheet production device 1 in Fig. 1. For example, the raw material container 21 for accommodating the culture solution A, the raw material container 22 for accommodating the culture solution B, the raw material container 23 for accommodating the culture solution C, the raw material container 24 for accommodating the culture solution D, the raw material container 25 for accommodating the culture solution E, and the raw material container 26 for accommodating the culture solution F are separately provided. The raw material containers 21, 22, 23, 24, 25, and 26 in Fig. 5 show a state before being attached to the cell sheet production device 1, and sterile connectors 81a, 83a, 84a, 85a, 86a, and 87a respectively constituting the sterile connectors 81, 83, 84, 85, 86, and 87 show a non-connected state.

The culture solution A, the culture solution B, the culture solution C, the culture solution E, and the culture solution F contain nutrients used for growing cells, and provide a growth environment of the cells. As the culture solution, a culture solution generally used for culturing cells can be used, and the culture solution can be appropriately determined depending on a type of the cells to be cultured. Examples thereof include a culture solution in which an inorganic salt, a carbohydrate, an amino acid, a vitamin, a protein, a peptide, collagen, a fatty acid, a lipid, a serum, or the like is contained in a buffer solution. Specifically, Dulbecco's modified Eagle medium (DMEM) can be used. The culture solutions A, B, C, E, and F may be culture solutions having the same composition or culture solutions having different compositions.

The culture solution D is a culture solution having a composition different from those of the other culture solutions A, B, C, E, and F. For example, the culture solution D contains a serum, a protein inhibitor, a growth factor, or other components, the composition of which is different from those of the other culture solutions A, B, C, E, and F in this regard.

The culture solutions A, B, C, D, E, and F are used as media for cell culture or used for medium exchange.

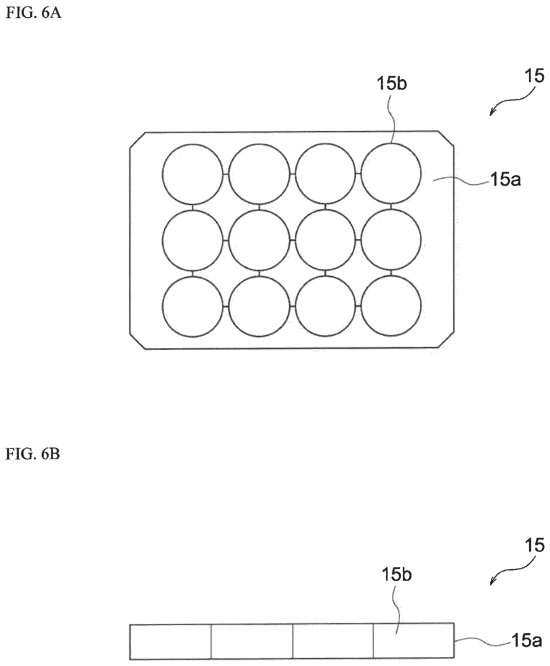

Fig. 6A shows a top view of the sheet forming container 15 incorporated in the cell sheet production device 1 in Fig. 1. Fig. 6B shows a side view of the sheet forming container 15 in Fig. 6A. The sheet forming container 15 is provided with a plurality of (3×4 in the drawing) sheet forming portions 15b arranged in a matrix on a substrate 15a. In the sheet forming container 15 shown in Fig. 6A, the number of sheet forming portions 15b provided in the sheet forming container 15 is 12, but the number of the sheet forming portions 15b is not particularly limited.

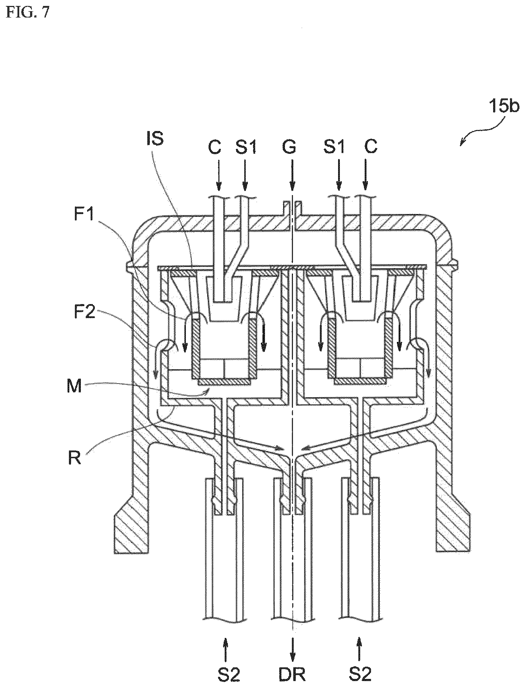

Fig. 7 shows a cross-sectional view of a part of the sheet forming portion 15b incorporated in the cell sheet production device 1 in Fig. 1. The number of sheet forming solution tanks IS may be one or more. In the sheet forming portion 15b shown in Fig. 7, a plurality of (for example, 2×2) sheet forming solution tanks IS are provided, and the drawing shows a cross section of two sheet forming solution tanks IS. The sheet forming solution tank IS is configured by a commercially available insert (product name: Transwell 3470), and a plane substrate for cell adhesion M is provided on a bottom surface thereof. A cell suspension C and a culture solution S1 are supplied to the sheet forming solution tank IS. Here, as the culture solution S1, a culture solution containing serum or the like and a culture solution not containing serum or the like can be appropriately selected and supplied. The sheet forming portion 15b shown in Fig. 7 is provided with four sheet forming solution tanks IS, and the drawing shows a cross section of two sheet forming solution tanks IS, but the number of the sheet forming solution tanks IS is not particularly limited, and the number of the sheet forming portions 15b is also not particularly limited.

A receiver tank R is provided in a lower portion of the sheet forming solution tank IS. A culture solution S2 is supplied to the receiver tank R. A flow rate of the culture solution S2 supplied to the receiver tank R is controllable, and a culture solution inside the receiver tank R is replaced when the cell sheet is produced.

A window portion is provided between the sheet forming solution tank IS and the receiver tank R, and an overflow F1 which occurs due to an unnecessary liquid component inside the sheet forming solution tank IS being discharged to the receiver tank R by a shaking operation or a discharging operation to be described later can be generated. Further, a window portion is provided on an outer peripheral side of the receiver tank R, and an overflow F2 which occurs due to the unnecessary liquid component inside the receiver tank R being discharged by a shaking operation or a discharge operation to be described later can be generated. The unnecessary liquid component discharged from the receiver tank R is discharged from the sheet forming portion 15b to the outside through a drain pipe DR.

The sheet forming portion 15b is provided with a pipe to which air G containing carbon dioxide is supplied. A concentration of the carbon dioxide in the air G is, for example, 4% to 6%, and the air is supplied from an air supply unit 19 such as a cylinder in which the air containing carbon dioxide is accommodated. The supply of the air G containing carbon dioxide promotes activation of the cells in the cell suspension entered the sheet forming solution tank IS. For example, a gas supplied to the sheet forming portion 15b may be air having a concentration of carbon dioxide adjusted to about 4% to 6% in advance, or may be a carbon dioxide gas of 100% or another gas component such as water vapor as long as the environment is suitable for the activation of the cells in the sheet forming solution tank IS.

For example, a filter for preventing bacteria from entering is provided in a supply path of the air G containing carbon dioxide.

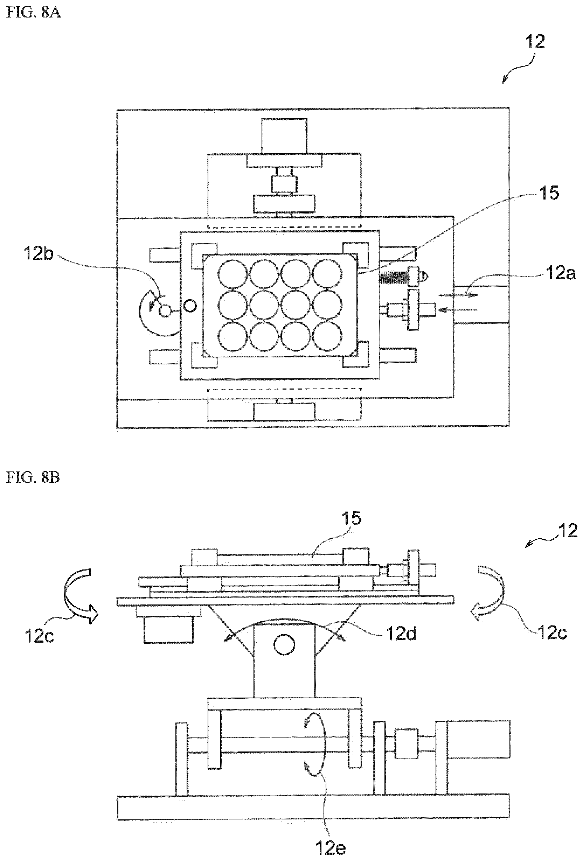

Fig. 8A shows a top view of a configuration in which the sheet forming container 15 of the cell sheet production device 1 in Fig. 1 is attached to the shaking device 12. Fig. 8B shows a side view of the configuration in which the sheet forming container 15 in Fig. 8A is attached to the shaking device 12. The sterile closed circuit for a cell sheet production device 100 is attached to the cell sheet production device 1 such that the sheet forming container 15 is fixed to the shaking device 12. The shaking device 12 is configured to be able to execute a shaking operation including, for example, a reciprocating operation 12a and a tapping operation 12b for applying an impact to the sheet forming container 15, a horizontal reciprocating rotation operation 12c, and inclining operations 12d and 12e for inclining the sheet forming container 15.

For example, the shaking device 12 includes an inclined portion and a tapping portion, the inclined portion inclines the sheet forming container 15 at an angle of 5 degrees to 45 degrees with respect to either a front-back direction or a left-right direction or both directions with respect to a horizontal plane at an interval of 0.1 seconds/times to 1 second/times, and the tapping portion being applies an impact of an acceleration of 1.0 G to 30 G at 1 second/times to 5 seconds/times to the sheet forming container 15.

Further, for example, the inclined portion is configured to be able to incline the sheet forming container 15 at an angle of 15 degrees to 60 degrees with respect to the horizontal plane in order to discharge the unnecessary liquid component inside the sheet forming container 15. Accordingly, the overflow F1 which occurs due to the unnecessary liquid component inside the sheet forming solution tank IS being discharged to the receiver tank R and the overflow F2 which occurs due to the unnecessary liquid component inside the receiver tank R being discharged can be generated by inclining the sheet forming container 15.

In the above-mentioned cell sheet production device 1, in order to confirm a connection error or the presence or absence of a leakage of each pipe, a pump or a pinch valve, and a pressure sensor may be provided in each pipe, and a mechanism for detecting a state in which a predetermined internal pressure is not reached by transferring air of a predetermined pressure into the pipe may be provided.

### (Method for Producing Cell Sheet)

A method for producing the cell sheet of the present embodiment will be described with reference to Fig. 1. First, the sterile closed circuit for a cell sheet production device 100 is attached to the cell sheet production device 1. That is, the washing container 13 is attached to the centrifuge 11, and the activation container 14 and the sheet forming container 15 are attached to the shaking device 12. The washing container 13, the activation container 14, the sheet forming container 15, the pipes connecting those containers, and the waste liquid container 16 maintain a closed system, and are subjected to sterilization in advance before use. The raw material containers 21, 22, 23, 24, 25, and 26 are attached to the raw material storage 20, and the frozen cell preservation container 31 is attached to the thawing device 30. The pipes are connected in a sterile state through the sterile connectors 81, 82, 83, 84, 85, 86, and 87. The washing container 13, the activation container 14, the sheet forming container 15, the pipes connecting those containers, and the waste liquid container 16 may be fixedly arranged using a panel or the like. Connection portions of the pipes may be marked or color-coded to simplify a connection work at the time of attachment.

After the sterile closed circuit for a cell sheet production device 100, the raw material containers 21, 22, 23, 24, 25, and 26, and the frozen cell preservation container 31 are attached to the cell sheet production device 1 and the pipes are connected, a connection failure and the presence or absence of leakage of each pipe are confirmed by a pump mechanism or the pinch valve provided in each pipe, or by pressurization and depressurization of each pipe in a case where a pressurization and depressurization mechanism is provided in each pipe.

Next, the frozen cells in the frozen cell preservation container 31 are thawed in the thawing device 30. Here, the frozen cells are obtained by freezing cells collected from a patient.

In the thawing device 30, for example, the frozen cell preservation container 31 is heated together to thaw the preserved frozen cells. A thawing temperature is, for example, 4°C to 37°C. Thereafter, a culture solution is injected into the frozen cell preservation container 31 from the raw material container 21 through the pipe 61, and is transferred to the washing container 13 together with the thawed cells.

Next, a cryoprotectant added to the cells is removed in the washing container 13. For example, the cells and the culture solution transferred to the washing container 13 are subjected to centrifugation by causing a rotator of the centrifuge 11 to which the washing container 13 is attached to rotate, and are separated into cells and a culture solution containing the cryoprotectant. The cells are transferred to the activation container 14 through the pipe 69 together with a culture solution for transfer. The culture solution containing the cryoprotectant is discharged to the waste liquid container 16 by a transfer device.

Next, activation of the cells is executed. The activation of the cells referred to herein is also referred to as preculture. For example, the cells transferred to the activation container 14 are diluted with a culture solution for activation injected from the raw material container 23 through the pipe 64, and left to stand until the cells are activated. A temperature of the activation process is 34°C to 40°C. In this case, in order to promote the activation of the cells, the activation container 14 may be shaken by the shaking device 12. A time for the activation process to be executed is about 3 days to 5 days, for example.

In a case where the cells are adherent cells, the cells adhere to the container when activated, so that a dispersion liquid such as trypsin is added to detach the cells from the container. Thereafter, in order to inactivate the trypsin, a culture solution or the like containing serum is added from the raw material container 24 and the raw material container 25 through the pipes 65, 65a, 66, and b. The culture solution containing the activated cells is transferred to the washing container 13 through the pipe 70, and separated into cells and a culture solution by centrifugation. The obtained activated cells are suspended with a culture solution for suspension to obtain a cell suspension, and the cell suspension is transferred to the sheet forming container 15 through the pipe 68.

In the present embodiment, the washing container 13 and the activation container 14 are used as separate containers, but may be used as a washing activation container by being integrated with each other. In this case, the above-mentioned transfer between the washing container 13 and the activation container 14 is omitted, and the steps are simplified.

In the above-mentioned washing step and activation step, washing and activation may be repeated a plurality of times as necessary. In this case, in the activation container 14, the activated cells may be suspended with a culture solution for suspension to obtain a cell suspension, and the cell suspension may be transferred to the sheet forming container 15 through the pipe 71.

The cell suspension transferred to the sheet forming container 15 is subjected to an optical inspection by the inspection unit 18, and it is inspected whether a cell density thereof is controlled to fall within the above-mentioned high density range, for example, 3,000 cells/mm² or more. In a case where the cell density is out of the above-mentioned range, the culture solution is discharged or added to adjust the cell concentration.

Next, the activated high-concentration cells are left to stand for a certain period of time on the plane substrate in the sheet forming container 15, and thus a cell sheet 80 is automatically produced. A temperature of the sheet forming process for the cells is 34°C to 40°C. In this case, in order to promote the sheet formation, the sheet forming container 15 may be shaken by the shaking device 12. A time for the sheet forming process of the cells to be executed is about 5 days, for example.

The cell sheet 80 has a flat shape, but may be a three-dimensional cell mass in a case where the cell sheet to be laminated has a thickness in a longitudinal direction.

In the shaking operation, for example, by driving the inclined portion of the shaking device 12, the sheet forming container 15 is inclined at an angle of 5 degrees to 45 degrees with respect to either the front-back direction or the left-right direction or both directions with respect to the horizontal plane at an interval of 0.1 seconds/times to 1 second/times. Further, by driving the tapping portion of the shaking device 12, an impact is applied to the sheet forming container 15.

In order to discharge the unnecessary liquid component inside the sheet forming container 15, the sheet forming container 15 may be inclined at an angle of 5 degrees to 100 degrees with respect to the horizontal plane.

Next, a groove portion having a depth of 0.1 mm to 0.5 mm is provided on the outer periphery of the side surface of the sheet forming container 15 such that the cell sheet produced in the sheet forming container 15 to a state suitable for surgery can be easily detached from the sheet forming container 15 without a tool, and by manually applying a force in the direction of separating from the upper portion and the lower portion of the groove, a space sufficient for detaching the cell sheet from the sheet forming container 15 appears, and the completed cell sheet is taken out from the sheet forming container 15 and used for a transplantation surgery.

The above-mentioned removal from the sheet forming container 15 can be executed by cutting a pipe in a sterile state at any position suitable for cutting the sterile closed circuit for a cell sheet production device 100 including the sheet forming container 15. The pipe may be cut by a sterile cutter or by thermal welding of the pipe.

The sterile closed circuit for a cell sheet production device 100 and the pipes and the like included therein are disposable and replaced with new ones for each use.

### (Operation and Effects of Cell Sheet Production Device)

According to the cell sheet production device 1 of the present embodiment, the cell sheet can be produced by an automatic operation, and the cell sheet can be produced by a device smaller than the CPC or the isolator system.

In particular, according to the cell sheet production device 1 of the present embodiment, it is possible to form a membrane (for example, Bruch's membrane) composed of an extracellular matrix on the surface of the cell sheet on a side opposite to the plane substrate for cell adhesion M on which the cell sheet is produced. In a case where the cell sheet on which the membrane composed of an extracellular matrix is formed is to be transplanted, the cell sheet can be transplanted without being turned over, which is advantageous for the transplantation surgery.

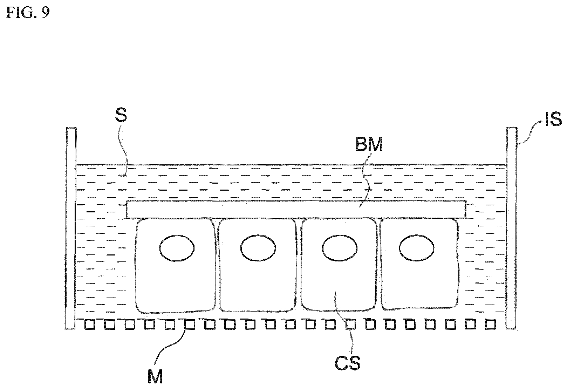

Fig. 9 shows a schematic diagram illustrating how a membrane BM composed of an extracellular matrix is formed on a surface of a cell sheet CS on the side opposite to the plane substrate for cell adhesion M. The plane substrate for cell adhesion M is provided on the bottom surface of the sheet forming solution tank IS. A mixed liquid S containing a cell suspension and a culture solution is accommodated in the sheet forming solution tank IS, and cell culture is executed to produce the cell sheet CS on the plane substrate for cell adhesion M. In this case, the membrane BM composed of an extracellular matrix is formed on the surface of the cell sheet CS on the side opposite to the plane substrate for cell adhesion M.

In the sterile closed circuit for a cell sheet production device 100, a pipe for transferring the cell suspension to the sheet forming container 15 and a pipe for supplying serum or the like are individually provided, and amounts and timings of supplying the cell suspension and the serum can be individually controlled. By the timing control, the stability of formation of the membrane composed of an extracellular matrix is improved.

By using a small automatic device capable of producing a cell sheet, the cell sheet can be produced automatically without depending on a skill of a manual work. The sterile closed circuit for a cell sheet production device used in the cell sheet production device can be replaced with a new circuit every time it is used, and the cell sheet for each patient can be efficiently produced while avoiding a risk of contamination. In addition, as the sterile closed circuit for a cell sheet production device, one circuit may be provided for one cell sheet production device, or a plurality of circuits may be installed for one cell sheet production device.

Since the cell sheet production device is a closed system, an installation portion of the cell sheet production device does not need to be a clean room, and since the cell sheet production device has a small size, it may be installed in a portion where a transplantation treatment is executed. Accordingly, a need to transport the cell sheet is eliminated, thereby reducing a risk of damage to the cell sheet.

The above-mentioned cell sheet production device 1 of the present embodiment can support treatment actions and the like that are difficult to cure particularly except for cell tissue transplantation, such as age-related macular degeneration.

### (First Example)

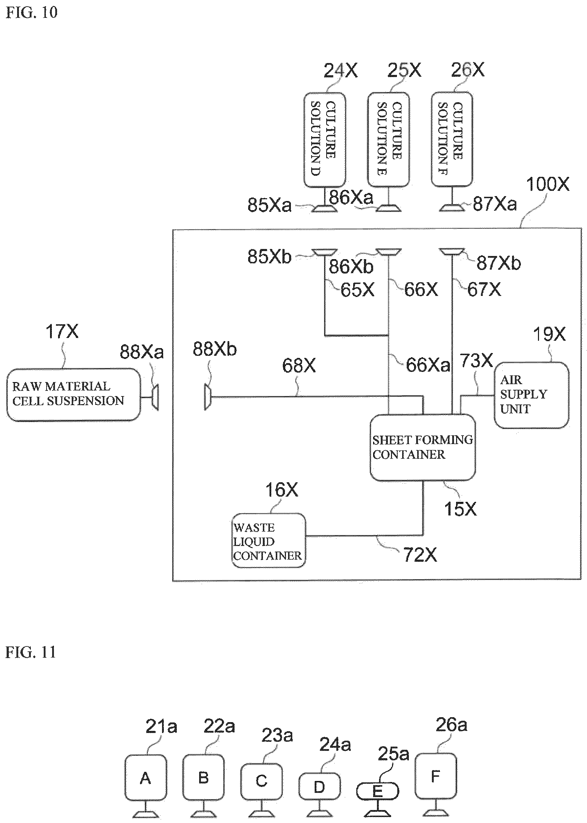

Fig. 10 schematically shows a configuration of a sheet forming container according to a first example. A sterile closed circuit for a cell sheet production device 100X includes a sheet forming container 15X and a waste liquid container 16X, and is provided with pipes 65X, 66X, 66Xa, 67X, 68X, and 72X connected to the containers. The pipe 66X is provided with a sterile connector 88Xb, and a raw material cell suspension 17X can be injected into the sheet forming container 15X by connecting a sterile connector 88Xa. The pipe 65X is provided with a sterile connector 85Xb, and a culture solution D can be injected from the raw material container 24X into the sheet forming container 15X by connecting a sterile connector 85Xa. The pipe 66X is provided with a sterile connector 86Xb, and a culture solution E can be injected from the raw material container 25X into the sheet forming container 15X by connecting a sterile connector 86Xa. The pipe 67X is provided with a sterile connector 87Xb, and a culture solution F can be injected from the raw material container 26X into the sheet forming container 15X by connecting a sterile connector 87Xa. The sheet forming container 15X is directly connected to an air supply unit 19X via an air supply pipe 73X to which a sterile filter is attached, or is indirectly connected to the air supply unit 19X via another tube. Air from the air supply unit 19X necessary for producing a cell sheet can be directly or indirectly injected into the sheet forming container 15X. The sterile filter is attached to the air supply pipe 73X, so that a sterile environment of air for culture in the thermostatic bath 10 can be maintained.

The sterile closed circuit for a cell sheet production device 100X of the present example is a circuit in which the washing container 13, the activation container 14, and the pipes connected to the containers are removed from the sterile closed circuit for a cell sheet production device 100 of the embodiment, and the pipe 68X is provided such that the cell suspension can be supplied from the outside. Except for the above, this example is the same as the embodiment.

The sterile closed circuit for a cell sheet production device 100X of the present example can be used in the cell sheet production device 1 of the above-mentioned embodiment, a cell sheet can be produced by an automatic operation, and the cell sheet can be produced by a device smaller than the CPC or the isolator system.

In a case where the sterile closed circuit for a cell sheet production device 100X of the present example is used in the cell sheet production device 1, containers corresponding to the washing container 13 and the activation container 14 are separately used. Since the sterile closed circuit for a cell sheet production device 100X does not include the washing container 13 and the activation container 14 from the beginning, the sheet forming container 15X after production of the cell sheet can be easily removed.

### (Second Example)

Fig. 11 schematically shows a configuration of raw material containers according to a second example. For example, a raw material container 21a for accommodating a culture solution A, a raw material container 22a for accommodating a culture solution B, a raw material container 23a for accommodating a culture solution C, a raw material container 24a for accommodating a culture solution D, a raw material container 25a for accommodating a culture solution E, and a raw material container 26a for accommodating a culture solution F are separately provided. Each of the raw material containers 21a, 22a, 23a, 24a, 25a, and 26a has a size capable of accommodating an amount suitable for one cell sheet production, and may be disposable and replaced with a new one for each use.

### (Third Example)

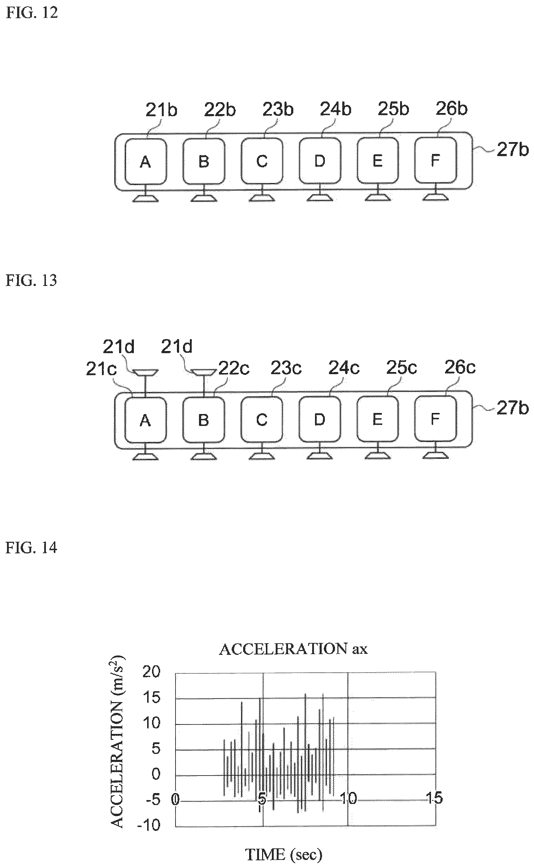

Fig. 12 schematically shows a configuration of raw material containers according to a third example. A raw material container 21b for accommodating a culture solution A, a raw material container 22b for accommodating a culture solution B, a raw material container 23b for accommodating a culture solution C, a raw material container 24b for accommodating a culture solution D, a raw material container 25b for accommodating a culture solution E, and a raw material container 26b for accommodating a culture solution F are collectively provided in one package 27b. The respective raw material containers 21b, 22b, 23b, 24b, 25b, and 26b are separated from each other in the package 27b and raw materials are not mixed with each other, and an arrangement order of the raw material containers is fixed, so that erroneous attachment of the raw material containers can be prevented.

### (Fourth Example)

Fig. 13 schematically shows a configuration of raw material containers according to a fourth example. A raw material container 21c for accommodating a culture solution A, a raw material container 22c for accommodating a culture solution B, a raw material container 23c for accommodating a culture solution C, a raw material container 24c for accommodating a culture solution D, a raw material container 25c for accommodating a culture solution E, and a raw material container 26c for accommodating a culture solution F are collectively provided in one package 27b. Further, the raw material container 21c and the raw material container 22c are each provided with a connection port 21d with a sterile connector. A gas is blown from the connection port 21d, and raw materials inside the raw material container 21c and the raw material container 22c can be extruded and used, and the raw materials can be used to the end without waste. The connection port 21d is provided in some or all of the raw material containers 21c, 22c, 23c, 24c, 25c, and 26c.

### (Fifth Example)

A sample of a frozen cell of a retinal pigment epithelial cell collected from an eye was subjected to thawing and activation by the cell sheet production device 1 described in the above-mentioned embodiment, and RPE cells obtained by treating the sample with 0.1% of trypsin and 0.02% of EDTA for 5 minutes were concentrated by centrifugation. The number of cells in the obtained cell suspension is measured, and 1,000,000 cells and 2,000,000 cells (cell densities of 5,000 cells/mm² and 10,000 cells/mm²) thereof were seeded in a sheet forming container (4-well chamber slide (Lab-Tek)), and the cells were cultured. As a result, a cell sheet of human retinal pigment epithelial cells can be formed on a plane substrate for cell adhesion.

The obtained cell sheet was subjected to immunostaining. The immunostaining was executed by fixing a sheet with 4% of paraformaldehyde, then executing whole mount or freezing or producing paraffin sections, executing staining by an enzyme antibody method or a fluorescent antibody method using a primary antibody against a target protein, such as elastin, collagen IV, collagen I, actin, cytokeratin, occludin, and ZO-1, and observing by an optical microscope or a fluorescent microscope. The actin can also be stained by fluorescently labeled phalloidin and observed. As a result of such immunostaining, an expression of intercellular adhesion and smooth muscle actin can be confirmed one day after seeding. Thereafter, an expression of the elastin and the collagen IV, which are components of the membrane (Bruch's membrane) constituted by the extracellular matrix, can be confirmed on a serum contact surface of the RPE cells.

The same results can be obtained by separately executing the immunostaining as described below. The produced cell sheet of human retinal pigment epithelial cells was washed twice with a 1M phosphate buffered saline (PBS), and fixed for 15 minutes in 0.2M PBS containing 4% of paraformaldehyde. After the fixation, the cell sheet was washed five times with a Tween 20-containing Tris-buffer saline (TBS-T). Next, after blocking the cell sheet with a protein blocking buffer, the cell sheet was incubated with a primary antibody at 4°C overnight. As the primary antibody to be used, an anti-occludin goat polyclonal antibody (1:100; Santa Cruz Biotechnology) or an anti-elastin rabbit polyclonal antibody (1:100; Calbiochem) was used. The cell sheet was washed three times with TBS-T, and then incubated with a secondary antibody at room temperature for 30 minutes. Next, the cell sheet was washed three times with TBS-T, dried, and then enclosed together with DAPI using vectashield. Each section can be observed with an optical microscope and a fluorescent microscope (AX-70; Olympus Optical Co., Ltd., Tokyo, Japan).

### (Sixth Example)

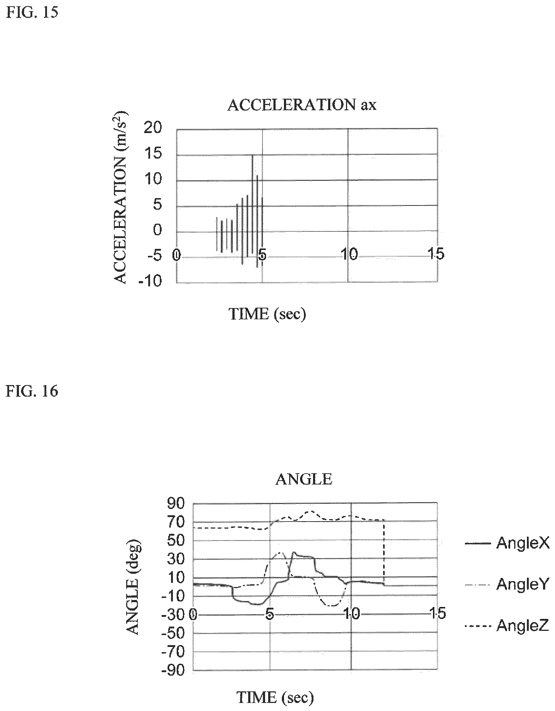

The shaking device 12 of the cell sheet production device 1 described in the above-mentioned embodiment was used to execute a tight inclining operation on the sheet forming container at an angle and an acceleration shown in Fig. 16 at inclining angles in vertical and horizontal directions simultaneously with a tight tapping operation for 5 seconds to 6 seconds shown in Fig. 14, thereby evenly seeding cells in a sheet forming container adjusted to a predetermined cell concentration on a plane substrate for cell adhesion to produce a homogeneous cell sheet.

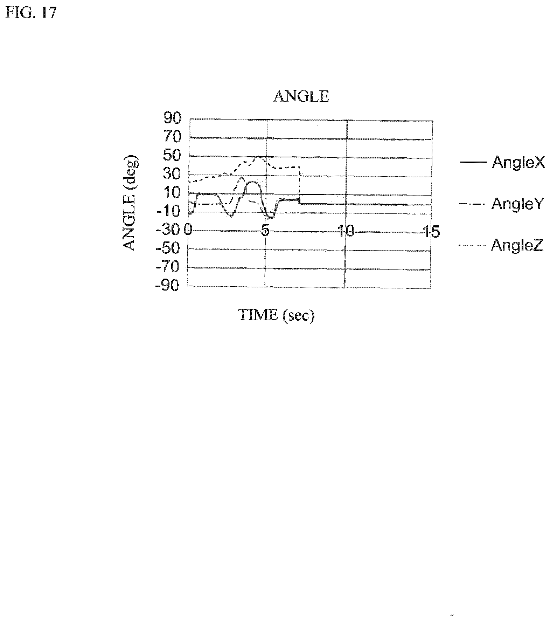

As a comparative example, in a case of a short and weak tapping operation of about 3 seconds shown in Fig. 15 and a short and weak inclining operation of about 3 seconds shown in Fig. 17, the cells in the sheet forming container adjusted to the predetermined cell concentration are not evenly seeded on the plane substrate for cell adhesion, and a homogeneous cell sheet is not produced.

As described above, according to the present example, by using the cell sheet production device 1 described in the above-mentioned embodiment, a stable and strong shaking operation can be executed as compared to the manual work, and a cell sheet can be produced while avoiding variation in quality without depending on the skill of the manual work.

### (Seventh Example)

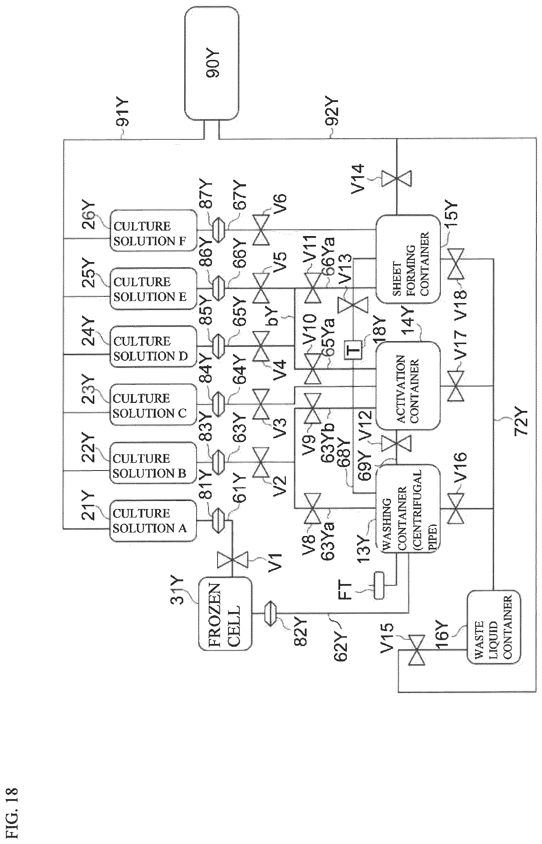

Fig. 18 schematically shows a configuration of a sterile closed circuit for a cell sheet production device, a raw material container, and pipes connecting the sterile closed circuit for a cell sheet production device and the raw material container in a cell sheet production device according to a seventh example. In the cell sheet production device according to the present example, instead of providing a tube (pellistor) pump in each pipe, a pressure control unit 90Y is connected to each of raw material containers 21Y, 22Y, 23Y, 24Y, 25Y, and 26Y, a frozen cell preservation container 31Y, a washing container 13Y, an activation container 14Y, and a sheet forming container 15Y. The pressure control unit 90Y is configured to generate a pressure difference between the respective containers and transfer a liquid. The pressure control unit 90Y executes pressurization by supply of air and depressurization by suction. A pipe 91Y is a pressurizing pipe, and a pipe 92Y is a depressurizing pipe. The pipe 91Y is connected to the raw material containers 21Y, 22Y, 23Y, 24Y, 25Y, and 26Y with a sterile filter attached thereto. The pipe 92Y is connected to the sheet forming container 15Y and the waste liquid container 16Y with a sterile filter attached thereto. The respective pipes connected to the respective containers are provided with pinch valves V1 to V6 and V8 to V18. The pressure control unit 90Y controls opening and closing of the pinch valves V1 to V6 and V8 to V18, and is provided to be able to adjust pressures inside each of the raw material containers 21Y, 22Y, 23Y, 24Y, 25Y, and 26Y, the frozen cell preservation container 31Y, the washing container 13Y, the activation container 14Y, and the sheet forming container 15Y, and the pipes connected to those containers. By opening and closing the pinch valves V1 to V6 and V8 to V18 and adjusting the pressure inside each of the raw material containers 21Y, 22Y, 23Y, 24Y, 25Y, and 26Y, the frozen cell preservation container 31Y, the washing container 13Y, the activation container 14Y, and the sheet forming container 15Y, and the pipes connected to those containers, a pressure difference is generated between specific containers, and a culture solution or a cell suspension is transferred between the containers. The pressure control unit 90Y may also serve as a control unit that controls a temperature and a shaking operation of the thermostatic bath 10, but in the present example, the pressure control unit 90Y is provided separately from the control unit.

In the cell sheet production device of the present example, a pinch valve which is opened so as to transfer a liquid between selected containers and control of pressurization and depressurization are shown in Table 1. Table 1 shows a transfer source container, a transfer destination container, an opened pinch valve, control (pressure control) of pressurization to the pipe 91Y, and depressurization to the pipe 92Y.

**[Table 1]**

| Transfer source | Transfer destination | Opened pinch valve | Pressure control |
|---|---|---|---|
| 21Y | 31Y, 13Y | V1 | Pressurization |
| 13Y | 16Y | V15, V16 | Depressurization |
| 22Y | 13Y | V2, V8 | Pressurization |
| 13Y | 14Y | V12, V17, V15 | Depressurization |
| 22Y | 14Y | V2, V9, V17, V15 | Pressurization /Depressurization |
| 23Y | 14Y | V3, V17, V15 | Pressurization /Depressurization |
| 24Y | 14Y | V4, V10, V17, V15 | Pressurization /Depressurization |
| 14Y | 16Y | V17, V15 | Depressurization |
| 14Y | 13Y | V3, V12 | Pressurization |
| 13Y | 15Y | V13, V14 | Depressurization |
| 24Y | 15Y | V4, V11, V14 | Pressurization /Depressurization |
| 25Y | 15Y | V5, V11, V14 | Pressurization /Depressurization |
| 26Y | 15Y | V6, V14 | Pressurization /Depressurization |
| 15Y | 16Y | V13, V18, V15 | Depressurization |

In a case where the liquid inside is transferred from the raw material container 21Y to the frozen cell preservation container 31Y and the washing container 13Y, the pinch valve V1 is opened and the pipe 91Y is pressurized.

In a case where the liquid inside is transferred from the washing container 13Y to the waste liquid container 16Y, the pinch valves V15 and V16 are opened, and the pipe 92Y is depressurized.

In a case where the liquid inside is transferred from the raw material container 22Y to the washing container 13Y, the pinch valves V2 and V8 are opened, and the pipe 91Y is pressurized.

In a case where the liquid inside is transferred from the washing container 13Y to the activation container 14Y, the pinch valves V12, V17, and V15 are opened, and the pipe 92Y is depressurized.

In a case where the liquid inside is transferred from the raw material container 22Y to the activation container 14Y, the pinch valves V2, V9, V17, and V15 are opened, the pipe 91Y is pressurized, and the pipe 92Y is depressurized.

In a case where the liquid inside is transferred from the raw material container 23Y to the activation container 14Y, the pinch valves V3, V17, and V15 are opened, the pipe 91Y is pressurized, and the pipe 92Y is depressurized.

In a case where the liquid inside is transferred from the raw material container 24Y to the activation container 14Y, the pinch valves V4, V10, V17, and V15 are opened, the pipe 91Y is pressurized, and the pipe 92Y is depressurized.

In a case where the liquid inside is transferred from the activation container 14Y to the waste liquid container 16Y, the pinch valves V17 and V15 are opened, and the pipe 92Y is depressurized.

In a case where the liquid inside is transferred from the activation container 14Y to the washing container 13Y, the pinch valves V3 and V12 are opened, and the pipe 91Y is pressurized.

In a case where the liquid inside is transferred from the washing container 13Y to the sheet forming container 15Y, the pinch valves V13 and V14 are opened, and the pipe 92Y is depressurized.

In a case where the liquid inside is transferred from the raw material container 24Y to the sheet forming container 15Y, the pinch valves V4, V11, and V14 are opened, the pipe 91Y is pressurized, and the pipe 92Y is depressurized.

In a case where the liquid inside is transferred from the raw material container 25Y to the sheet forming container 15Y, the pinch valves V5, V11, and V14 are opened, the pipe 91Y is pressurized, and the pipe 92Y is depressurized.

In a case where the liquid inside is transferred from the raw material container 26Y to the sheet forming container 15Y, the pinch valves V6 and V14 are opened, the pipe 91Y is pressurized, and the pipe 92Y is depressurized.

In a case where the liquid inside is transferred from the sheet forming container 15Y to the waste liquid container 16Y, the pinch valves V13, V18, and V15 are opened, and the pipe 92Y is depressurized.

### (Eighth Example)

In an eighth example, an example is shown in which the liquid inside is transferred from a selected transfer source container to each transfer destination container under conditions shown in Table 2 using the cell sheet production device according to the seventh example. A transfer amount of the liquid, a pipe inner diameter and a pipe length related to the transfer, a supply pressure to the pipe 91Y or the pipe 92Y, and liquid transfer results are shown in Table 2.

**[Table 2]**

| Transfer | Transfer | Transfer | Transfer | Pipe | Pipe | Supply pressure | Liquid transfer |
|---|---|---|---|---|---|---|---|
| position | source | destination | amount ml | inner diameter mm | length mm | kPa | result |
| P1 | 21Y | 31Y, 13Y | 30 | 1.0 | 605 | More than 0 and less than 10 | × With liquid residue |
| | | | | | | 10 or more and 20 or less | o Total amount transferred (90 seconds or less) |
| | | | | | | More than 20 | × Ejection too strong |
| P2 | 13Y | 14Y | 10.8 | 1.0 | 460 | More than -5 and less than 0 | × With liquid residue |
| | | | | | | -18 or more and -5 or less | o Total amount transferred (60 seconds or less) |
| | | | | | | Less than -18 | × Ejection too strong |
| P3 | 14Y | 13Y | 20 | 1.0 | 460 | More than -1 and less than 0 | × With liquid residue |
| | | | | | | -20 or more and -1 or less | o Total amount transferred (60 seconds or less) |
| | | | | | | Less than -20 | × Ejection too strong |
| P4 | 13Y | 16Y | 30 | 3.2 | 955 | More than -2 and less than 0 | × With liquid residue |
| | | | | | | -4 or more and -2 or less | o Total amount transferred (15 seconds or less) |
| | | | | | | Less than -4 | × Ejection too strong |
| P5 | 23Y | 14Y | 70 | 1.0 | 525 | More than 0 and less than 10 | × With liquid residue |
| | | | | | | 10 or more and 20 or less | o Total amount transferred (120 seconds or less) |
| | | | | | | More than 20 | × Ejection too strong |
| P6 | 14Y | 16Y | 70 | 3.2 | 625 | More than -2.5 and less than 0 | × With liquid residue |
| | | | | | | -5 or more and -2.5 or less | o Total amount transferred (25 seconds or less) |
| | | | | | | Less than -5 | × Ejection too strong |
| P7 | 13Y | 15Y | 0.8 | 1.0 | 680 | More than -10 and less than 0 | × With liquid residue |
| | | | | | | -20 or more and -10 or less | ○ Total amount transferred (11 seconds or less) |
| | | | | | | Less than -20 | × Ejection too strong |
| P8 | 26Y | 15Y | 0.5 | 1.0 | 480 | More than 0 and less than 3 | × With liquid residue |
| | | | | | | 3 or more and 5 or less | o Total amount transferred (5 seconds or less) |
| | | | | | | More than 5 | × Ejection too strong |

At a transfer position P1, the culture solution was transferred from the raw material container 21Y to the washing container 13Y via the frozen cell preservation container 31Y. The transfer amount was 30 ml, the inner diameter of the pipe connecting the raw material container 21Y to the frozen cell preservation container 31Y and the washing container 13Y was 1.0 mm, and the pipe length was 605 mm. When the supply pressure to the pipe 91Y was 10 kPa or more and 20 kPa or less, the total amount of the liquid can be transferred within 90 seconds or less. When the supply pressure to the pipe 91Y was more than 0 kPa and less than 10 kPa, the pressure was too weak, the liquid cannot be transferred, and there was a liquid residue. When the supply pressure to the pipe 91Y was more than 20 kPa, the pressure was too high, and the liquid was strongly ejected into the transfer destination container, which may cause a damage to the cells.

At a transfer position P2, the cell suspension was transferred from the washing container 13Y to the activation container 14Y. The transfer amount was 10.8 ml, the inner diameter of the pipe connecting the washing container 13Y and the activation container 14Y was 1.0 mm, and the pipe length was 460 mm. When the supply pressure to the pipe 92Y was -18 kPa or more and -5 kPa or less, the total amount of the liquid can be transferred within 60 seconds or less. When the supply pressure to the pipe 92Y was more than -5 kPa and less than 0 kPa, the pressure was too weak, the liquid cannot be transferred, and there was a liquid residue. When the supply pressure to the pipe 92Y was less than -18 kPa, the pressure was too high, and the liquid was strongly ejected into the transfer destination container, which may cause a damage to the cells.

At a transfer position P3, the cell suspension was transferred from the activation container 14Y to the washing container 13Y. The transfer amount was 20 ml, the inner diameter of the pipe connecting the activation container 14Y and the washing container 13Y was 1.0 mm, and the pipe length was 460 mm. When the supply pressure to the pipe 92Y was -20 kPa or more and -1 kPa or less, the total amount of the liquid can be transferred within 60 seconds or less. When the supply pressure to the pipe 92Y was more than -1 kPa and less than 0 kPa, the pressure was too weak, the liquid cannot be transferred, and there was a liquid residue. When the supply pressure to the pipe 92Y was less than -20 kPa, the pressure was too high, and the liquid was strongly ejected into the transfer destination container, which may cause a damage to the cells.

At a transfer position P4, an unnecessary liquid component was transferred from the washing container 13Y to the waste liquid container 16Y. The transfer amount was 30 ml, the inner diameter of the pipe connecting the washing container 13Y and the waste liquid container 16Y was 3.2 mm, and the pipe length was 955 mm. When the supply pressure to the pipe 92Y was -4 kPa or more and -2 kPa or less, the total amount of the liquid can be transferred within 15 seconds or less. When the supply pressure to the pipe 92Y was more than -2 kPa and less than 0 kPa, the pressure was too weak, the liquid cannot be transferred, and there was a liquid residue. When the supply pressure to the pipe 92Y was less than -4 kPa, the pressure was too high, and the liquid was strongly ejected into the transfer destination container.

At a transfer position P5, the culture solution was transferred from the raw material container 23Y to the activation container 14Y. The transfer amount was 70 ml, the inner diameter of the pipe connecting the raw material container 23Y and the activation container 14Y was 1.0 mm, and the pipe length was 525 mm. When the supply pressure to the pipe 91Y was 10 kPa or more and 20 kPa or less, the total amount of the liquid can be transferred within 120 seconds or less. When the supply pressure to the pipe 91Y was more than 0 kPa and less than 10 kPa, the pressure was too weak, the liquid cannot be transferred, and there was a liquid residue. When the supply pressure to the pipe 91Y was more than 20 kPa, the pressure was too high, and the liquid was strongly ejected into the transfer destination container, which may cause a damage to the cells.

At a transfer position P6, an unnecessary liquid component was transferred from the activation container 14Y to the waste liquid container 16Y. The transfer amount was 70 ml, the inner diameter of the pipe connecting the activation container 14Y and the waste liquid container 16Y was 3.2 mm, and the pipe length was 625 mm. When the supply pressure to the pipe 92Y was -5 kPa or more and -2.5 kPa or less, the total amount of the liquid can be transferred within 25 seconds or less. When the supply pressure to the pipe 92Y was more than -2.5 kPa and less than 0 kPa, the pressure was too weak, the liquid cannot be transferred, and there was a liquid residue. When the supply pressure to the pipe 92Y was less than -5 kPa, the pressure was too high, and the liquid was strongly ejected into the transfer destination container.

At a transfer position P7, the cell suspension was transferred from the washing container 13Y to the sheet forming container 15Y. The transfer amount was 0.8 ml, the inner diameter of the pipe connecting the washing container 13Y and the sheet forming container 15Y was 1.0 mm, and the pipe length was 680 mm. When the supply pressure to the pipe 92Y was -20 kPa or more and -10 kPa or less, the total amount of the liquid can be transferred within 11 seconds or less. When the supply pressure to the pipe 92Y was more than -10 kPa and less than 0 kPa, the pressure was too weak, the liquid cannot be transferred, and there was a liquid residue. When the supply pressure to the pipe 92Y was less than -20 kPa, the pressure was too high, and the liquid was strongly ejected into the transfer destination container, which may cause a damage to the cells.

At a transfer position P8, the culture solution was transferred from the raw material container 26Y to the sheet forming container 15Y. The transfer amount was 0.5 ml, the inner diameter of the pipe connecting the raw material container 26Y and the sheet forming container 15Y was 1.0 mm, and the pipe length was 480 mm. When the supply pressure to the pipe 91Y was 3 kPa or more and 5 kPa or less, the total amount of the liquid can be transferred within 5 seconds or less. When the supply pressure to the pipe 91Y was more than 0 kPa and less than 3 kPa, the pressure was too weak, the liquid cannot be transferred, and there was a liquid residue. When the supply pressure to the pipe 91Y was more than 5 kPa, the pressure was too high, and the liquid was strongly ejected into the transfer destination container, which may cause a damage to the cells.

As described above, based on the results at the transfer positions P1 to P8, it is confirmed that a total amount of the liquid can be transferred between the selected containers within a predetermined time by appropriately controlling the supply pressures to the pipe 91Y and the pipe 92Y.

In the above-mentioned embodiments and examples, the following various modifications can be made without changing the gist of the present invention.

The above-mentioned embodiments and examples can be appropriately combined, and can be appropriately modified without departing from the gist of the invention. For example, the washing container and the activation container may be integrated with each other or may be separated from each other, and may be configured to be included in or not included in the sterile closed circuit. In addition, for example, the raw material containers may have a size capable of accommodating an amount suitable for one cell sheet production, may be collectively provided in one package, and may be replaced with a new one for each use in a disposable manner.

### Reference Sign List

1: cell sheet production device
10: thermostatic bath
11: centrifuge
12: shaking device
13: washing container
14: activation container
15: sheet forming container
16: waste liquid container
18: inspection unit
19: air supply unit
20: raw material storage
21, 22, 23, 24, 25, 26: raw material container
30: thawing device
31: frozen cell preservation container
40: local heating mechanism
50: control unit
51: display unit
61, 62, 63, 63a, 63b, 64, 65, 65a, 66, 66a, b, 67, 68, 69, 70, 71, 72: pipe
73: air supply pipe
81, 82, 83, 84, 85, 86, 87: sterile connector
100: sterile closed circuit for a cell sheet production device

## Claims

1. A cell sheet production device, to which a sterile closed circuit for a cell sheet production device is to be attached, the sterile closed circuit including
a sheet forming container provided with a sheet forming solution tank having a plane substrate for cell adhesion for producing a cell sheet,
a first pipe that is connected to the sheet forming container and injects a cell suspension in which cells are suspended into the sheet forming container,
a second pipe that is connected to the sheet forming container and injects a culture solution for medium exchange into the sheet forming container, and
a third pipe that is connected to the sheet forming container and discharges an unnecessary liquid component inside the sheet forming container,
the cell sheet production device comprising:
a concentration unit that is connected to the sheet forming container and concentrates the cell suspension, wherein
a concentration of the cell suspension is concentrated in the concentration unit, the concentrated cell suspension is injected into the sheet forming container through the first pipe, the cells are seeded on the plane substrate for cell adhesion at a cell seeding density of 3,000 cells/mm² or more, the unnecessary liquid component is discharged from the third pipe, and the culture solution for medium exchange is injected from the second pipe, thereby producing a cell sheet on the plane substrate for cell adhesion.

2. The cell sheet production device according to claim 1, wherein
the cell sheet production device and the sterile closed circuit for a cell sheet production device are configured to be detachable without a tool.

3. The cell sheet production device according to claim 1, further comprising:
a shaking device including an inclined portion and a tapping portion, the inclined portion inclines the sheet forming container at an angle of 5 degrees to 45 degrees with respect to either a front-back direction or a left-right direction or both directions with respect to a horizontal plane at an interval of 0.1 seconds/times to 1 second/times, and the tapping portion applies an impact of an acceleration of 1.0 G to 30 G at 1 time/s to 5 times/s to the sheet forming container.

4. The cell sheet production device according to claim 1, wherein
the sterile closed circuit for a cell sheet production device further includes a washing container that washes the cells constituting the cell suspension and an activation container that activates the cells.

5. The cell sheet production device according to claim 1, further comprising:
a thawing device that thaws frozen cells to obtain the cells constituting the cell suspension.

6. The cell sheet production device according to claim 1, further comprising:
at least one or all of a raw material storage that stores a container for accommodating a culture solution to be supplied to the sterile closed circuit for a cell sheet production device, a local heating mechanism that heats at least a part of the second pipe, and a thermostatic bath that keeps the sheet forming container at a predetermined temperature.

7. The cell sheet production device according to claim 6, wherein
the sterile closed circuit for a cell sheet production device further includes a washing container that washes the cells constituting the cell suspension and an activation container that activates the cells, and the thermostatic bath keeps the washing container and the activation container at the predetermined temperature.

8. The cell sheet production device according to claim 1, further comprising:
an inspection unit that inspects a cell density of the cell suspension to be injected into the sheet forming container.

9. The cell sheet production device according to claim 1, wherein
a container for accommodating a culture solution to be supplied to the sterile closed circuit for a cell sheet production device and the second pipe are connected through a sterile connector.

10. A cell sheet production device, to which a sterile closed circuit for a cell sheet production device is to be attached, the sterile closed circuit including
a sheet forming container provided with a sheet forming solution tank having a plane substrate for cell adhesion for producing a cell sheet,
a first pipe that is connected to the sheet forming container and injects a cell suspension in which cells are suspended into the sheet forming container,
a second pipe that is connected to the sheet forming container and injects a culture solution for medium exchange into the sheet forming container, and
a third pipe that is connected to the sheet forming container and discharges an unnecessary liquid component inside the sheet forming container,
the cell sheet production device comprising:
a concentration unit that is connected to the sheet forming container and concentrates the cell suspension;
a plurality of switching valves provided in a pipe connected to the sheet forming container, the pipe including the first pipe, the second pipe, and the third pipe; and
a liquid transferring mechanism that controls switching of the switching valves, and adjusts a pressure inside the sterile closed circuit for a cell sheet production device via a sterile filter, and transfers a liquid inside the sterile closed circuit for a cell sheet production device based on a difference in the pressure, wherein
a concentration of the cell suspension is concentrated in the concentration unit, the concentrated cell suspension is injected into the sheet forming container through the first pipe, the cells are seeded on the plane substrate for cell adhesion at a cell seeding density of 3,000 cells/mm² or more, the unnecessary liquid component is discharged from the third pipe, and the culture solution for medium exchange is injected from the second pipe, thereby producing a cell sheet on the plane substrate for cell adhesion.

11. A sterile closed circuit for a cell sheet production device, the sterile closed circuit comprising:
a sheet forming container provided with a sheet forming solution tank having a plane substrate for cell adhesion for producing a cell sheet;
a first pipe that is connected to the sheet forming container and injects a cell suspension in which cells are suspended into the sheet forming container;
a second pipe that is connected to the sheet forming container and injects a culture solution for medium exchange into the sheet forming container; and
a third pipe that is connected to the sheet forming container and discharges an unnecessary liquid component inside the sheet forming container, wherein
a concentration unit that concentrates the cell suspension is connected to the sheet forming container, and
a concentration of the cell suspension is concentrated in the concentration unit, the concentrated cell suspension is injected into the sheet forming container through the first pipe, the cells are seeded on the plane substrate for cell adhesion at a cell seeding density of 3,000 cells/mm² or more, the unnecessary liquid component is discharged from the third pipe, and the culture solution for medium exchange is injected from the second pipe, thereby producing a cell sheet on the plane substrate for cell adhesion.

12. The sterile closed circuit for a cell sheet production device according to claim 11, wherein
a cell sheet production device and the sterile closed circuit for a cell sheet production device are configured to be detachable without a tool.

13. The sterile closed circuit for a cell sheet production device according to claim 11, further comprising:
a washing container that washes the cells constituting the cell suspension and an activation container that activates the cells.

14. The sterile closed circuit for a cell sheet production device according to claim 11, wherein
a container for accommodating a culture solution to be supplied to the sterile closed circuit for a cell sheet production device and the second pipe are connected through a sterile connector.

15. A sterile closed circuit for a cell sheet production device to be attached to a cell sheet production device to produce a cell sheet, the sterile closed circuit comprising:
a sheet forming container provided with a sheet forming solution tank having a plane substrate for cell adhesion for producing a cell sheet;
a first pipe that is connected to the sheet forming container and injects a cell suspension in which cells are suspended into the sheet forming container;
a second pipe that is connected to the sheet forming container and injects a culture solution for medium exchange into the sheet forming container; and
a third pipe that is connected to the sheet forming container and discharges an unnecessary liquid component inside the sheet forming container, wherein
a concentration unit that concentrates the cell suspension is connected to the sheet forming container,
a plurality of switching valves are provided in a pipe connected to the sheet forming container, the pipe including the first pipe, the second pipe, and the third pipe,
a liquid transferring mechanism that controls switching of the switching valves, and adjusts a pressure inside the sterile closed circuit for a cell sheet production device via a sterile filter, and transfers a liquid inside the sterile closed circuit for a cell sheet production device based on a difference in the pressure is provided, and
a concentration of the cell suspension is concentrated in the concentration unit, the concentrated cell suspension is injected into the sheet forming container through the first pipe, the cells are seeded on the plane substrate for cell adhesion at a cell seeding density of 3,000 cells/mm² or more, the unnecessary liquid component is discharged from the third pipe, and the culture solution for medium exchange is injected from the second pipe, thereby producing a cell sheet on the plane substrate for cell adhesion.
